# EUROPEAN PATENT APPLICATION

(11) **EP 4 752 135 A1**
(43) Date of publication of application: **03.06.2026**
(21) Application number: 24845265.8
(22) Date of filing: 20.06.2024
(51) Int. Cl.: C07C 269/00, C07C 271/22, C07D 233/80

(54) **METHOD FOR PRODUCING AMIDE COMPOUND**

(30) Priority: 25.07.2023 JP 2023121036
(71) Applicant: Nippon Soda Co., Ltd., Tokyo 100-7010 (JP)
(72) Inventor: MISAKA, Remi, Takaoka-shi, Toyama 933-8507 (JP); TAKEHISA, Katsuma, Odawara-shi, Kanagawa 250-0280 (JP); NODA, Kaoru, Takaoka-shi, Toyama 933-8507 (JP)
(74) Representative: Mewburn Ellis LLP
(86) International application number: PCT/JP2024/022355
(87) International publication number: WO 2025/022883

(57) **Abstract**

A method for producing a compound represented by formula (2), the method including chemically reacting a compound represented by formula (3) with a compound represented by formula (4) to obtain a compound represented by formula (1); and hydrolyzing the obtained compound represented by the formula (1): 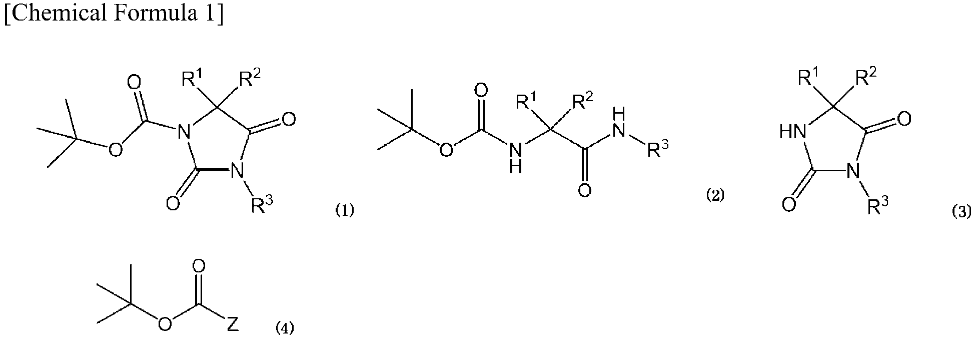 (in the formulas (1) to (4), R¹ and R² each independently represent a hydrogen atom, a substituted or unsubstituted C₁₋₆ alkyl group, or the like; R³ represents a substituted or unsubstituted C₁₋₆ alkyl group, or the like; and Z represents a t-butoxycarbonyloxy group, a halogeno group, or a 1H-imidazol-1-yl group.)

## Description

### TECHNICAL FIELD

The present invention relates to a method for producing an amide compound. More specifically, the present invention relates to a method for producing an N-BOC amino acid amide compound.

Priority is claimed on Japanese Patent Application No. 2023-121036, filed July 25, 2023, the content of which is incorporated herein by reference.

### BACKGROUND ART

N-BOC amino acid amide compounds and amino acid amide compounds are useful compounds as intermediates and the like for the production of medicines and agricultural chemicals. For example, Patent Document 1 discloses that in a production example of a compound 1-1 having acaricidal activity, t-butyl(2-methyl-1-oxo-1-((2,2,2-trifluoroethyl)amino)propan-2-yl)carbamate (compound 17: N-BOC amino acid amide compound) was obtained by a condensation reaction between an N-BOC amino acid (compound 8) and 2,2,2-trifluoroethylamine, and then 2-amino-2-methyl-N-(2,2,2-trifluoroethyl)propanamide hydrochloride (compound 18: amino acid amide compound) was obtained via a deprotection reaction.

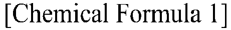

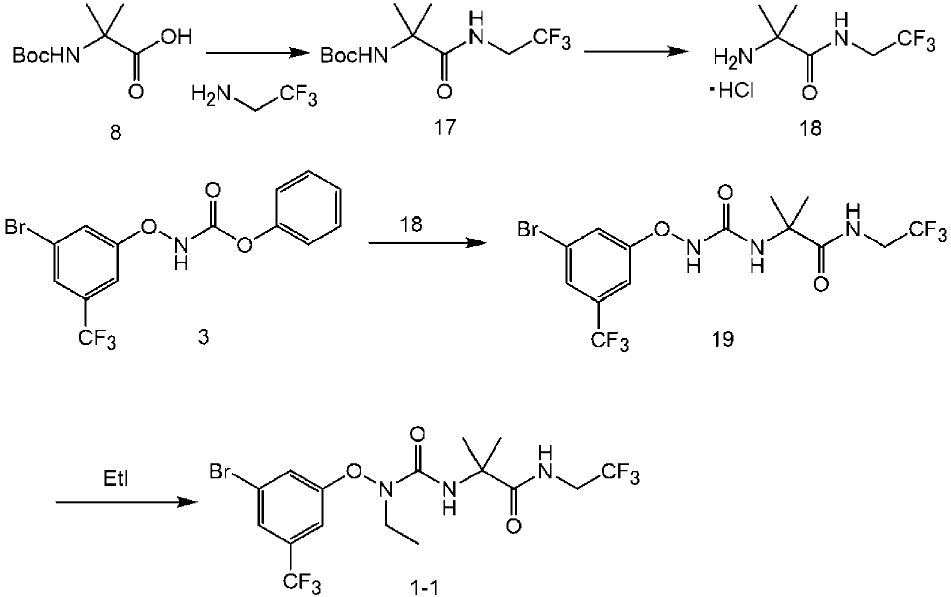

### Citation List

### Patent Document

Patent Document 1: International Patent Publication No. 2019/198592

### SUMMARY OF INVENTION

### Technical Problem

An object of the present invention is to provide a novel method for producing an N-BOC amino acid amide compound.

### Solution to Problem

As a result of intensive research in order to achieve the above object of the present invention, the present invention including the following aspects has been completed.
[1] A method for producing a compound represented by formula (2), the method including hydrolyzing a compound represented by formula (1): 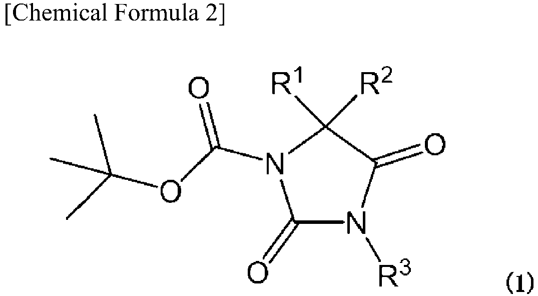
   in the formula (1), R¹ and R² each independently represent a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₃₋₆ cycloalkyl group, a substituted or unsubstituted phenyl group, or a substituted or unsubstituted 5- or 6-membered heteroaryl group; and R¹ and R² may together form a substituted or unsubstituted C₂₋₅ alkylene group,
   R³ represents a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₃₋₆ cycloalkyl group, a substituted or unsubstituted phenyl group, or a substituted or unsubstituted 5- or 6-membered heteroaryl group, 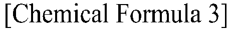 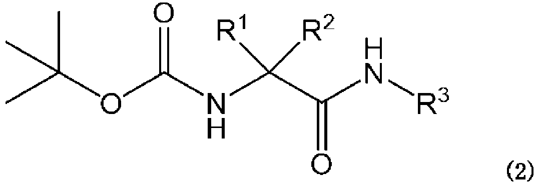
   in the formula (2), R¹, R², and R³ are the same as those defined in the formula (1).
[2] The method according to [1], further including chemically reacting a compound represented by formula (3) with a compound represented by formula (4) to obtain the compound represented by the formula (1), 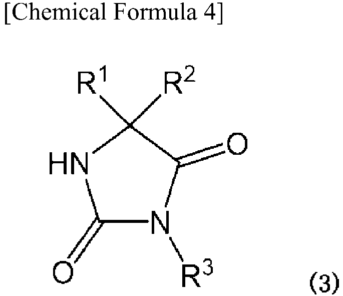 in the formula (3), R¹, R², and R³ are the same as those defined in the formula (1), 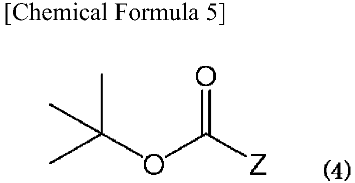 in the formula (4), Z represents a t-butoxycarbonyloxy group, a halogeno group, or a 1H-imidazol-1-yl group.
[3] The method according to [1], further including chemically reacting a compound represented by formula (3) with phosgene, diphosgene, or triphosgene to obtain a compound represented by formula (5), and
   chemically reacting the compound represented by the formula (5) with an alkali metal t-butoxide to obtain the compound represented by the formula (1), 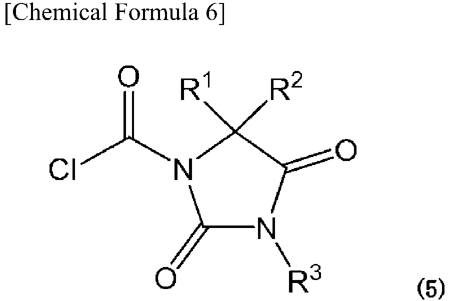
   in the formula (5), R¹, R², and R³ are the same as those defined in the formula (1).
[4] The method according to [2] or [3], further including chemically reacting a compound represented by formula (6) with a compound represented by formula (7) to obtain the compound represented by the formula (3), 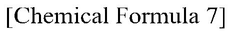 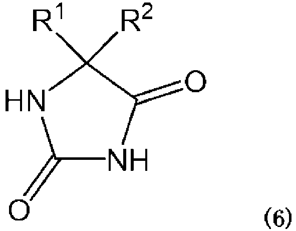 in the formula (6), R¹ and R² are the same as those defined in the formula (1), 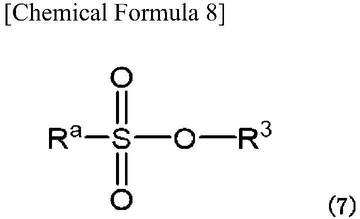 in the formula (7), R³ is the same as that defined in the formula (1). R^{a} represents a methyl group, a trifluoromethyl group, a phenyl group, or a tolyl group.

### Advantageous Effects of Invention

Hydrolysis of hydantoin typically yields glycine. Similarly, hydrolysis of 5-substituted hydantoins yields various amino acids.

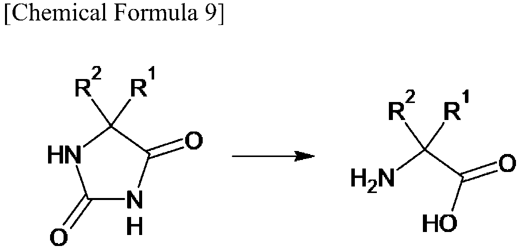

Further, in compounds in which a methoxycarbonyl group, ethoxycarbonyl group, n-propoxycarbonyl group, or i-propoxycarbonyl group is bonded to an amino group (compounds represented by formula (1a)), the following chemical reaction is allowed to proceed.

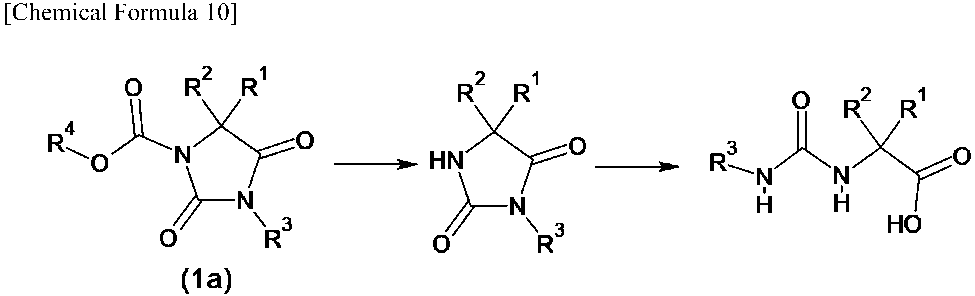

In the formula (1a), R⁴ represents a methyl group, an ethyl group, an n-propyl group, or an i-propyl group.

On the other hand, according to the production method of the present invention, regioselective hydrolysis is allowed to proceed due to the influence of a tert-butoxycarbonyl group (BOC group: a group known as a group to protect amino groups) in the compound represented by the formula (1), making it possible to obtain the compound represented by the formula (2) in high yield. The corresponding amino acid amide compound can be obtained when the compound represented by the formula (2) undergoes a deprotection reaction. The amino acid amide compound obtained by the production method of the present invention is useful as an intermediate for producing active ingredients of medicines and agricultural chemicals, such as a compound 1-1.

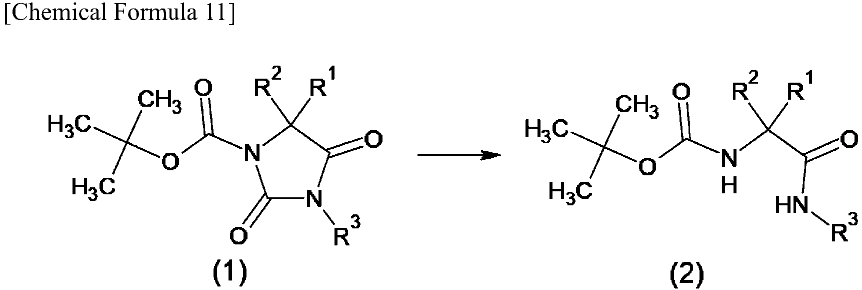

### DESCRIPTION OF EMBODIMENTS

One embodiment of the production method of the present invention involves converting a compound represented by formula (1) (hereinafter sometimes referred to as a compound (1)) into a compound represented by formula (2) (hereinafter sometimes referred to as a compound (2)).

Another embodiment of the production method of the present invention involves converting a compound represented by formula (3) (hereinafter sometimes referred to as a compound (3)) into the compound (1) and converting the compound (1) into the compound (2).

Another embodiment of the production method of the present invention involves converting a compound represented by formula (6) (hereinafter sometimes referred to as a compound (6)) into the compound (3), converting the compound (3) into the compound (1), and converting the compound (1) into the compound (2).

In the present invention, the term "unsubstituted" means that it is composed only of a group which becomes a mother nucleus. When it is described only by the name of the group which becomes the mother nucleus without being described as "substituted", it means "unsubstituted" unless otherwise stated.

On the other hand, the term "substituted" means that any hydrogen atom of a group which becomes a mother nucleus is substituted with a group (substituent) having the same or different structure as that of the mother nucleus. Therefore, a "substituent" is another group bonded to a group which becomes a mother nucleus. The number of substituents may be one, or two or more. The two or more substituents may be the same or different.

The terms "C₁₋₆" and the like mean that the number of carbon atoms in the group which becomes a mother nucleus is 1 to 6, and so on. The number of carbon atoms does not include the number of carbon atoms present in the substituent. For example, a butyl group having an ethoxy group as a substituent is classified as a C₂ alkoxy C₄ alkyl group.

A "substituent" is not particularly limited as long as it is chemically acceptable and has the effects of the present invention.

Hereinafter, groups which can be a "substituent" are exemplified.
A C₁₋₆ alkyl group such as a methyl group, an ethyl group, an n-propyl group, an i-propyl group, an n-butyl group, an s-butyl group, an i-butyl group, a t-butyl group, an n-pentyl group, and an n-hexyl group;
a C₂₋₆ alkenyl group such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;
a C₂₋₆ alkynyl group such as an ethynyl group, a 1-propynyl group, a 2-propynyl group, a 1-butynyl group, a 2-butynyl group, a 3-butynyl group, and a 1-methyl-2-propynyl group;
a C₃₋₆ cycloalkyl group such as a cyclopropyl group, a cyclobutyl group, a cyclopentyl group and a cyclohexyl group;
a phenyl group;
a 3- to 6-membered heterocyclyl group;
a C₁₋₆ alkoxy group such as a methoxy group, an ethoxy group, an n-propoxy group, an i-propoxy group, an n-butoxy group, an s-butoxy group, an i-butoxy group, and a t-butoxy group;
a C₆₋₁₀ aryloxy group such as a phenoxy group and a naphthoxy group;
a 5- to 6-membered heteroaryloxy group such as a thiazolyloxy group and a pyridyloxy group;
a C₁₋₆ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group;
a halogeno group such as a fluoro group, a chloro group, a bromo group, and an iodo group;
a C₁₋₆ haloalkyl group such as a chloromethyl group, a chloroethyl group, a trifluoromethyl group, a 1,2-dichloro-n-propyl group, a 1-fluoro-n-butyl group and a perfluoro-n-pentyl group;
a C₁₋₆ haloalkoxy group such as a trifluoromethoxy group, a 2-chloro-n-propoxy group and a 2,3-dichlorobutoxy group;
a formylamino group;
a C₁₋₆ alkylcarbonylamino group such as an acetylamino group, a propanoylamino group, a butyrylamino group and an i-propylcarbonylamino group;
a C₁₋₆ alkoxycarbonylamino group such as a methoxycarbonylamino group, an ethoxycarbonylamino group, an n-propoxycarbonylamino group and an i-propoxycarbonylamino group;
an unsubstituted or substituted aminocarbonyl group such as an aminocarbonyl group, a dimethylaminocarbonyl group, a phenylaminocarbonyl group and an N-phenyl-N-methylaminocarbonyl group;
a C₁₋₆ alkylthio group such as a methylthio group, an ethylthio group, an n-propylthio group, an i-propylthio group, an n-butylthio group, an i-butylthio group, an s-butylthio group and a t-butylthio group;
a C₁₋₆ haloalkylthio group such as a trifluoromethylthio group and a 2,2,2-trifluoroethylthio group;
a C₁₋₆ alkylsulfonyl group such as a methylsulfonyl group, an ethylsulfonyl group and a t-butylsulfonyl group;
a C₁₋₆ haloalkylsulfonyl group such as a trifluoromethylsulfonyl group and a 2,2,2-trifluoroethylsulfonyl group;
a cyano group; and a nitro group.

Further, in these "substituents", any hydrogen atom in the substituent may be substituted with a group having a different structure. Examples of the "substituent" in this case include a C₁₋₆ alkyl group, a C₁₋₆ haloalkyl group, a C₁₋₆ alkoxy group, a C₁₋₆ haloalkoxy group, a halogeno group, a cyano group and a nitro group.

Further, the above-described "3- to 6-membered heterocyclyl group" includes 1 to 4 hetero atoms selected from the group consisting of a nitrogen atom, an oxygen atom and a sulfur atom as constituent atoms of the ring. The heterocyclyl group may be either monocyclic or polycyclic. As long as the polycyclic heterocyclyl group includes at least one heterocyclic ring, the remaining ring may be any of a saturated alicyclic ring, an unsaturated alicyclic ring or an aromatic ring. Examples of the "3- to 6-membered heterocyclyl group" include a 3- to 6-membered saturated heterocyclyl group, a 5- to 6-membered heteroaryl group and a 5- to 6-membered partially unsaturated heterocyclyl group.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridazinyl group and a triazinyl group.

Examples of the 5-membered partially unsaturated heterocyclyl group include a pyrrolinyl group, a dihydrofuranyl group, an imidazolinyl group, a pyrazolinyl group, an oxazolinyl group, and an isoxazolinyl group.

Examples of the 6-membered partially unsaturated heterocyclyl group include a dihydropyranyl group.

### [Conversion of compound (1) to compound (2) (chemical reaction III)]

The compound (2) obtained by one embodiment of the production method of the present invention is a compound represented by the formula (2).

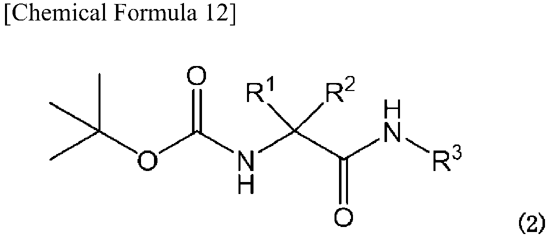

In the formula (2), R¹, R², and R³ are the same as those defined in the formula (1).

A substrate used in the chemical reaction III is the compound (1).

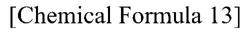

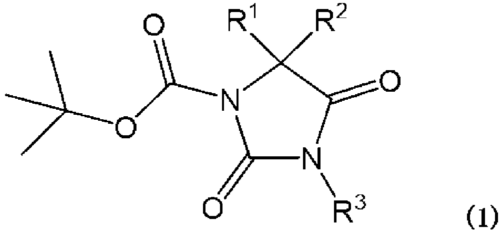

In the formula (1), R¹ and R² each independently represent a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₃₋₆ cycloalkyl group, a substituted or unsubstituted phenyl group, or a substituted or unsubstituted 5- or 6-membered heteroaryl group; and R¹ and R² may together form a substituted or unsubstituted C₂₋₅ alkylene group.

In the formula (1), R³ represents a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₃₋₆ cycloalkyl group, a substituted or unsubstituted phenyl group, or a substituted or unsubstituted 5- or 6-membered heteroaryl group.

The "C₁₋₆ alkyl group" represented by R¹ to R³ may be linear or branched. Examples of the "C₁₋₆ alkyl group" represented by R¹ to R³ include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group and an i-hexyl group.

Examples of the "C₂₋₆ alkenyl group" represented by R¹ to R³ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group.

Examples of the "C₂₋₆ alkynyl group" represented by R¹ to R³ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group.

As the substituent on the "C₁₋₆ alkyl group," "C₂₋₆ alkenyl group," or "C₂₋₆ alkynyl group" represented by R¹ to R³, a halogeno group such as a fluoro group, a chloro group, a bromo group and an iodo group; a hydroxyl group; a C₁₋₆ alkoxy group such as a methoxy group, an ethoxy group, an n-propoxy group, an i-propoxy group, an n-butoxy group, an s-butoxy group, an i-butoxy group and a t-butoxy group; a C₁₋₆ haloalkoxy group such as a 2-chloro-n-propoxy group, a 2,3-dichlorobutoxy group and a trifluoromethoxy group; a phenyl group; a phenyl group substituted with a halogeno group, C₁₋₆ haloalkyl group or C₁₋₆ haloalkoxy group, such as a 4-chlorophenyl group, a 4-trifluoromethylphenyl group and a 4-trifluoromethoxyphenyl group; or a cyano group is preferred.

Examples of the "C₃₋₆ cycloalkyl group" represented by R¹ to R³ include a cyclopropyl group, a cyclobutyl group, a cyclopentyl group and a cyclohexyl group.

The "5- to 6-membered heteroaryl group" represented by R¹ to R³ is a 5-membered ring or 6-membered ring group containing 1, 2, 3 or 4 hetero atoms selected from the group consisting of a nitrogen atom, an oxygen atom and a sulfur atom as constituent atoms of the ring. When there are two or more hetero atoms, they may be the same or different.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridazinyl group and a triazinyl group.

As the substituent on the "C₃₋₆ cycloalkyl group," "phenyl group," or "5- or 6-membered heteroaryl group" represented by R¹ to R³, a halogeno group such as a fluoro group, a chloro group, a bromo group and an iodo group; a C₁₋₆ alkyl group such as a methyl group, an ethyl group, an n-propyl group, an i-propyl group, an n-butyl group, an s-butyl group, an i-butyl group, a t-butyl group, an n-pentyl group and an n-hexyl group; a C₁₋₆ haloalkyl group such as a chloromethyl group, a chloroethyl group, a trifluoromethyl group, a 1,2-dichloro-n-propyl group and a 1-fluoro-n-butyl group; a hydroxyl group; a C₁₋₆ alkoxy group such as a methoxy group, an ethoxy group, an n-propoxy group, an i-propoxy group, an n-butoxy group, an s-butoxy group, an i-butoxy group and a t-butoxy group; a C₁₋₆ haloalkoxy group such as a 2-chloro-n-propoxy group, a 2,3-dichlorobutoxy group and a trifluoromethoxy group; or a cyano group; is preferred.

Examples of the C₂₋₅ alkylene group formed by R¹ and R² together include a dimethylene group, a trimethylene group, a tetramethylene group and a pentamethylene group. As the substituent on the "C₂₋₅ alkylene group", a halogeno group such as a fluoro group, a chloro group, a bromo group, and an iodo group is preferred.

In the present embodiment, R¹ and R² are preferably substituted or unsubstituted C₁₋₆ alkyl groups, and examples thereof include a methyl group.

In the present embodiment, R³ is preferably a substituted or unsubstituted C₁₋₆ alkyl group, and examples thereof include a 2,2,2-trifluoroethyl group.

By the chemical reaction III, the N-C bond between positions 1 and 2 and the N-C bond between positions 2 and 3 in the compound (1) are broken, thereby allowing conversion to the compound (2). Carbon dioxide is produced during the conversion to the compound (2).

The chemical reaction III can be carried out, for example, in a solvent in the presence of an inorganic base.

Examples of the inorganic base that can be used in the chemical reaction III include sodium carbonate, sodium bicarbonate, potassium carbonate, potassium bicarbonate, sodium hydroxide, potassium hydroxide, sodium hydride, and potassium hydride.

The amount of the inorganic base used is, for example, from 0.5 to 10 moles, preferably from 1.0 to 7.0 moles, with respect to 1 mole of the compound (1).

In the chemical reaction III, depending on the solvent, any of an aqueous phase composed of water only, a homogeneous phase composed of water and an organic solvent, or a heterogeneous phase composed of water and an organic solvent may be formed as a reaction site.

The organic solvent that can be used in the chemical reaction III is not particularly limited as long as it is inert with respect to the chemical reaction III.

Examples of the organic solvent for forming the homogeneous phase include alcohols such as methanol, ethanol, n-propanol, i-propanol, and n-butanol; acetone, dimethyl sulfoxide (abbreviated name: DMSO), and dimethylformamide (abbreviated name: DMF).

Examples of the organic solvent for forming the heterogeneous phase include ethers such as diethyl ether, diisopropyl ether, diethylene glycol dimethyl ether (product name: diglyme), tetrahydrofuran (abbreviated name: THF), 4-methyltetrahydropyran (abbreviated name: MTHP), and dioxane; halogenated hydrocarbons such as dichloromethane, chloroform, and 1,2-dichloroethane (abbreviated name: DCE); aliphatic hydrocarbons such as pentane, hexane, cyclohexane, heptane, and octane; and aromatic hydrocarbons such as toluene and xylene.

In any case, one type of these organic solvents can be used alone or two or more types thereof can be used in combination.

The amount of the solvent used in the chemical reaction III is not particularly limited, but is preferably from 1 to 100 parts by mass with respect to 1 part by mass of the compound (1).

One embodiment of the chemical reaction III involves dissolving an inorganic base in a solvent and adding the compound (1) thereto to complete the hydrolysis.

Addition of the compound (1) to the reaction site can be carried out all at once or gradually. Dropwise addition is suitable for gradual addition. Addition of the compound (1) to the reaction site is preferably carried out while stirring. The temperature during addition (dropwise addition) of the compound (1) is preferably from 0°C to 15°C. The temperature during the hydrolysis reaction after completion of the addition is not particularly limited, but is preferably the temperature during addition (dropwise addition), more preferably from 0°C to 15°C.

The pressure during the hydrolysis reaction is, for example, from 0.1 to 1 MPa, preferably from 0.1 to 0.5 MPa, and more preferably 0.1 MPa. The time required for the hydrolysis reaction is not particularly limited, but is, for example, from 0.5 to 24 hours. The chemical reaction III is preferably carried out under an inert gas atmosphere.

After completion of the chemical reaction III, post-treatments such as extraction with an organic solvent, and drying and concentration of the obtained organic layer can be performed. Furthermore, if necessary, a product in the chemical reaction III (compound (2)) may be purified by operations such as recrystallization and chromatography.

Although the compound (1) as the substrate used in the chemical reaction III is not particularly limited by its production method, for example, it can be obtained by a method including conversion from the compound (3) to the compound (1).

### [Conversion of compound (3) to compound (1) (chemical reaction II-1)]

The compound (3) as the substrate in the chemical reaction II-1 is a compound represented by the formula (3).

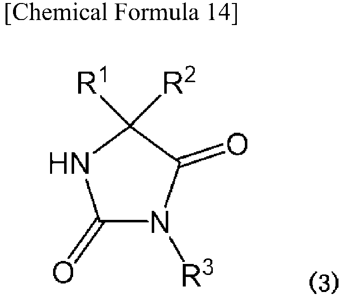

In the formula (3), R¹, R², and R³ are the same as those defined in the formula (1).

A compound (4) is used in the chemical reaction II-1. The compound (4) is a compound represented by formula (4).

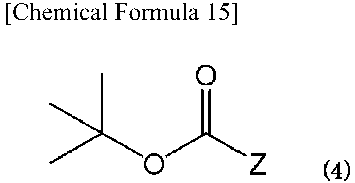

In the formula (4), Z represents a t-butoxycarbonyloxy group, a halogeno group, or a 1H-imidazol-1-yl group.

In the present embodiment, Z is preferably a t-butoxycarbonyloxy group or a halogeno group.

Examples of the "halogeno group" represented by Z include a fluoro group, a chloro group, a bromo group, and an iodo group. Of these, a chloro group is preferred.

The compound (4) used in the present embodiment may be synthesized by oneself through a known method, or may be synthesized through some form of method and made commercially available by others.

### [When the compound (4) is di-t-butyl dicarbonate (chemical reaction II-1A)]

A compound in which Z in the formula (4) is a t-butoxycarbonyloxy group is dit-butyl dicarbonate (English name: di-tert-butyl dicarbonate; abbreviated name:
(Boc)₂O).

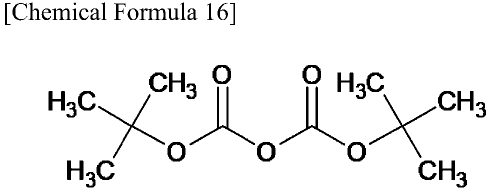

When (Boc)₂O is used as the compound (4), the chemical reaction II-1 can be carried out in an organic solvent in the presence of an organic base.

Examples of the organic base that can be used in the chemical reaction II-1A include triethylamine, tetramethylethylenediamine, N,N-diisopropylethylamine, N,N-dimethylaniline, N,N-diethylaniline, 4-methylmorpholine, 1-azabicyclo[2.2.2]octane, 1,4-diazabicyclo[2.2.2]octane (abbreviated name: DABCO), 1,8-diazabicyclo[5.4.0]undec-7-ene, 1,5-diazabicyclo[4.3.0]non-5-ene, pyridine, 4-(dimethylamino)pyridine, and 2,6-dimethylpyridine.

The amount of (Boc)₂O used in the chemical reaction II-1A is, for example, preferably from 1.0 to 2.0 moles, and more preferably from 1.0 to 1.5 moles, with respect to 1 mole of the compound (3).

The amount of the organic base used in the chemical reaction II-1A is, for example, preferably from 2.0 to 5.0 moles, and more preferably from 2.0 to 3.0 moles, with respect to 1 mole of the compound (3).

The organic solvent that can be used in the chemical reaction II-1A is not particularly limited as long as it is inert with respect to the chemical reaction II-1A. Examples of the organic solvent that can be used in the chemical reaction II-1A include ethers such as diethyl ether, diisopropyl ether, diethylene glycol dimethyl ether (product name: diglyme), tetrahydrofuran (abbreviated name: THF), 4-methyltetrahydropyran (abbreviated name: MTHP), and dioxane; halogenated hydrocarbons such as dichloromethane, chloroform, and 1,2-dichloroethane (abbreviated name: DCE); aliphatic hydrocarbons such as pentane, hexane, cyclohexane, heptane, and octane; aromatic hydrocarbons such as toluene and xylene; esters such as methyl acetate, ethyl acetate, propyl acetate, butyl acetate, dimethyl carbonate, and diethyl carbonate; ketones such as acetone, anone, and methyl isobutyl ketone (abbreviated name: MIBK); and other solvents such as dimethyl sulfoxide (abbreviated name: DMSO) and dimethylformamide (abbreviated name: DMF). One type of these organic solvents can be used alone or two or more types thereof can be used in combination. The amount of the organic solvent used is not particularly limited, but is preferably from 1 to 100 parts by mass with respect to 1 part by mass of the compound (3).

One embodiment of the chemical reaction II-1A involves dissolving the compound (3) and an organic base in an organic solvent and adding (Boc)₂O thereto to complete the chemical reaction.

The addition of (Boc)₂O is preferably carried out gradually in order to suppress a sudden temperature rise due to the heat of reaction. The addition rate of (Boc)₂O may be adjusted by monitoring the temperature inside a reactor. The temperature during the addition of (Boc)₂O is not particularly limited and may be, for example, room temperature. The temperature during the chemical reaction II-1A is not particularly limited and may be the temperature during the addition or a higher temperature. For example, it is from room temperature to 80°C. The pressure during the chemical reaction II-1A is, for example, from 0.1 to 1 MPa, preferably from 0.1 to 0.5 MPa, and more preferably 0.1 MPa. The time required for the chemical reaction II-1A is not particularly limited and is, for example, from 0.5 to 24 hours. The chemical reaction II-1A is preferably carried out under an inert gas atmosphere.

After completion of the chemical reaction II-1A, post-treatments such as extraction with an organic solvent, and drying and concentration of the obtained organic layer can be performed. Furthermore, if necessary, a product in the chemical reaction II-1A may be purified by operations such as recrystallization and chromatography.

### [When the compound (4) is t-butyl haloformate (chemical reaction II-1B)]

A compound in which Z in the formula (4) is a halogeno group is t-butyl haloformate (English name: tert-butyl haloformate).

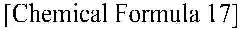

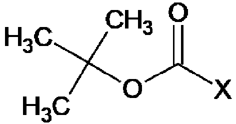

X is a halogeno group.

When t-butyl haloformate is used as the compound (4), the chemical reaction II-1B can be carried out in an organic solvent in the presence of an inorganic base.

Examples of the inorganic base that can be used in the chemical reaction II-1B include sodium carbonate, sodium bicarbonate, potassium carbonate, potassium bicarbonate, sodium hydroxide, potassium hydroxide, sodium hydride, and potassium hydride.

The amount of t-butyl haloformate used in the chemical reaction II-1B is, for example, preferably from 1.0 to 3.0 moles, and more preferably from 1.5 to 2.0 moles, with respect to 1 mole of the compound (3). The amount of the inorganic base used in the chemical reaction II-1B is, for example, preferably from 2.0 to 5.0 moles, and more preferably from 3.0 to 4.0 moles, with respect to 1 mole of the compound (3).

The organic solvent that can be used in the chemical reaction II-1B is not particularly limited as long as it is inert with respect to the chemical reaction II-1B. Examples of the organic solvent that can be used in the chemical reaction II-1B include ethers such as diethyl ether, diisopropyl ether, diethylene glycol dimethyl ether (product name: diglyme), tetrahydrofuran (abbreviated name: THF), 4-methyltetrahydropyran (abbreviated name: MTHP), and dioxane; halogenated hydrocarbons such as dichloromethane, chloroform, and 1,2-dichloroethane (abbreviated name: DCE); aliphatic hydrocarbons such as pentane, hexane, cyclohexane, heptane, and octane; aromatic hydrocarbons such as toluene and xylene; esters such as methyl acetate, ethyl acetate, propyl acetate, butyl acetate, dimethyl carbonate, and diethyl carbonate; ketones such as acetone, anone, and methyl isobutyl ketone (abbreviated name: MIBK); and other solvents such as dimethyl sulfoxide (abbreviated name: DMSO) and dimethylformamide (abbreviated name: DMF). One type of these organic solvents can be used alone or two or more types thereof can be used in combination. The amount of the organic solvent used is not particularly limited, but is preferably from 1 to 100 parts by mass with respect to 1 part by mass of the compound (3).

One embodiment of the chemical reaction II-1B involves dissolving the compound (3) in an organic solvent and adding an inorganic base and t-butyl haloformate thereto to complete the chemical reaction.

The addition of t-butyl haloformate is preferably carried out gradually in order to suppress a sudden temperature rise due to the heat of reaction. The addition rate of t-butyl haloformate may be adjusted by monitoring the temperature inside a reactor. The temperature during the addition of t-butyl haloformate is preferably from -10°C to 5°C. The temperature during the chemical reaction II-1B is not particularly limited and may be the temperature during the addition, and for example, from -10°C to 10°C. The pressure during the chemical reaction II-1B is, for example, from 0.1 to 1 MPa, preferably from 0.1 to 0.5 MPa, and more preferably 0.1 MPa. The time required for the chemical reaction II-1B is not particularly limited and is, for example, from 0.5 to 24 hours. The chemical reaction II-1B is preferably carried out under an inert gas atmosphere.

After completion of the chemical reaction II-1B, the reaction liquid is added to an acidic aqueous solution to neutralize any remaining inorganic base. Then, post-treatments such as extraction with an organic solvent, and drying and concentration of the obtained organic layer can be performed. Furthermore, if necessary, a product in the chemical reaction II-1B may be purified by operations such as recrystallization and chromatography.

### [When the compound (4) is t-butyl 1H-imidazole-1-carboxylate (chemical reaction II-1C)]

A compound in which Z in the formula (4) is a 1H-imidazol-1-yl group is t-butyl 1H-imidazole-1-carboxylate (English name: tert-butyl 1H-imidazol-1-carboxylate).

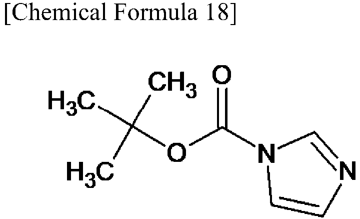

When t-butyl 1H-imidazole-1-carboxylate is used as the compound (4), the chemical reaction II-1 can be carried out in an organic solvent.

The amount of t-butyl 1H-imidazole-1-carboxylate used in the chemical reaction II-1C is, for example, preferably from 1.0 to 2.0 moles, and more preferably from 1.0 to 1.5 moles, with respect to 1 mole of the compound (3).

The organic solvent that can be used in the chemical reaction II-1C is not particularly limited as long as it is inert with respect to the chemical reaction II-1C. Examples of the organic solvent that can be used in the chemical reaction II-1C include ethers such as diethyl ether, diisopropyl ether, diethylene glycol dimethyl ether (product name: diglyme), tetrahydrofuran (abbreviated name: THF), 4-methyltetrahydropyran (abbreviated name: MTHP), and dioxane; halogenated hydrocarbons such as dichloromethane, chloroform, and 1,2-dichloroethane (abbreviated name: DCE); aliphatic hydrocarbons such as pentane, hexane, cyclohexane, heptane, and octane; aromatic hydrocarbons such as toluene and xylene; esters such as methyl acetate, ethyl acetate, propyl acetate, butyl acetate, dimethyl carbonate, and diethyl carbonate; ketones such as acetone, anone, and methyl isobutyl ketone (abbreviated name: MIBK); and other solvents such as dimethyl sulfoxide (abbreviated name: DMSO) and dimethylformamide (abbreviated name: DMF). One type of these organic solvents can be used alone or two or more types thereof can be used in combination. The amount of the organic solvent used is not particularly limited, but is preferably from 1 to 100 parts by mass with respect to 1 part by mass of the compound (3).

One embodiment of the chemical reaction II-1C involves dissolving the compound (3) in an organic solvent and adding t-butyl 1H-imidazole-1-carboxylate thereto to complete the chemical reaction.

The addition of t-butyl 1H-imidazole-1-carboxylate is preferably carried out gradually in order to suppress a sudden temperature rise due to the heat of reaction. The addition rate of t-butyl 1H-imidazole-1-carboxylate may be adjusted by monitoring the temperature inside a reactor. The temperature during the addition of t-butyl 1H-imidazole-1-carboxylate is not particularly limited and may be room temperature. The temperature during the chemical reaction II-1C is not particularly limited and may be the temperature during the addition or a higher temperature. For example, it is from room temperature to 80°C. The pressure during the chemical reaction II-1C is, for example, from 0.1 to 1 MPa, preferably from 0.1 to 0.5 MPa, and more preferably 0.1 MPa. The time required for the chemical reaction II-1C is not particularly limited and is, for example, from 0.5 to 24 hours. The chemical reaction II-1C is preferably carried out under an inert gas atmosphere.

After completion of the chemical reaction II-1C, post-treatments such as extraction with an organic solvent, and drying and concentration of the obtained organic layer can be performed. Furthermore, if necessary, a product in the chemical reaction II-1C may be purified by operations such as recrystallization and chromatography.

### [Conversion of compound (3) to compound (1) (chemical reaction II-2)]

The chemical reaction II-2 is a two-stage reaction.

First, the compound (3) is subjected to a chemical reaction with phosgene, diphosgene, or triphosgene (chemical reaction II-2a). As a result, a compound represented by formula (5) (hereinafter sometimes referred to as a compound (5)) is obtained.

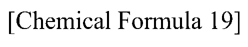

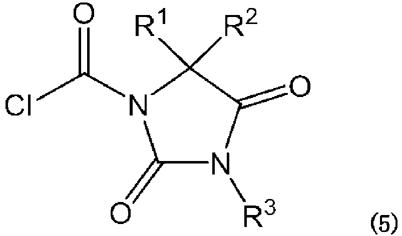

In the formula (5), R¹, R², and R³ are the same as those defined in the formula (1).

Subsequently, the compound (5) is subjected to a chemical reaction with an alkali metal t-butoxide (chemical reaction II-2b). As a result, the compound (1) is obtained.

The chemical reaction II-2a can be carried out in an organic solvent in the presence of an organic base.

Examples of the organic base that can be used in the chemical reaction II-2a include triethylamine, tetramethylethylenediamine, N,N-diisopropylethylamine, N,N-dimethylaniline, N,N-diethylaniline, 4-methylmorpholine, 1-azabicyclo[2.2.2]octane, 1,4-diazabicyclo[2.2.2]octane (abbreviated name: DABCO), 1,8-diazabicyclo[5.4.0]undec-7-ene, 1,5-diazabicyclo[4.3.0]non-5-ene, pyridine, 4-(dimethylamino)pyridine, and 2,6-dimethylpyridine.

The amount of phosgene used in the chemical reaction II-2a is, for example, preferably from 1.0 to 2.0 moles, and more preferably from 1.2 to 1.5 moles, with respect to 1 mole of the compound (3).

The amount of diphosgene used in the chemical reaction II-2a is, for example, preferably from 0.5 to 1.0 moles, and more preferably from 0.6 to 0.8 moles, with respect to 1 mole of the compound (3).

The amount of triphosgene used in the chemical reaction II-2a is, for example, preferably from 0.35 to 0.7 moles, and more preferably from 0.5 to 0.6 moles, with respect to 1 mole of the compound (3).

Any one of phosgene, diphosgene, or triphosgene may be used alone or any two or more of them may be used in combination. 1 mole of diphosgene becomes 2 moles of phosgene by decomposition. 1 mole of triphosgene becomes 3 moles of phosgene by decomposition. When using a combination of any two or more of phosgene, diphosgene, or triphosgene, the amount of phosgene can be set, for example, preferably from 0.5 to 1.0 moles, and more preferably from 0.6 to 0.8 moles, with respect to 1 mole of the compound (3), taking into account the phosgene that will be obtained by decomposition. The amount of the organic base used in the chemical reaction II-2a is, for example, preferably from 2.0 to 5.0 moles, and more preferably from 2.0 to 3.0 moles, with respect to 1 mole of the compound (3).

The organic solvent that can be used in the chemical reaction II-2a is not particularly limited as long as it is inert with respect to the chemical reaction II-2a. Examples of the organic solvent that can be used in the chemical reaction II-2a include ethers such as diethyl ether, diisopropyl ether, diethylene glycol dimethyl ether (product name: diglyme), tetrahydrofuran (abbreviated name: THF), 4-methyltetrahydropyran (abbreviated name: MTHP), and dioxane; halogenated hydrocarbons such as dichloromethane, chloroform, and 1,2-dichloroethane (abbreviated name: DCE); aliphatic hydrocarbons such as pentane, hexane, cyclohexane, heptane, and octane; aromatic hydrocarbons such as toluene and xylene; esters such as methyl acetate, ethyl acetate, propyl acetate, butyl acetate, dimethyl carbonate, and diethyl carbonate; and ketones such as acetone, anone, and methyl isobutyl ketone (abbreviated name: MIBK). One type of these organic solvents can be used alone or two or more types thereof can be used in combination. The amount of the organic solvent used is not particularly limited, but is preferably from 1 to 100 parts by mass with respect to 1 part by mass of the compound (3).

One embodiment of the chemical reaction II-2a involves dissolving the compound (3) and phosgene, diphosgene, or triphosgene in an organic solvent and adding an organic base thereto to complete the chemical reaction.

The addition of organic base is preferably carried out gradually in order to suppress a sudden temperature rise due to the heat of reaction. The addition rate of the organic base may be adjusted by monitoring the temperature inside a reactor. The temperature during the addition of the organic base is preferably from -10°C to 10°C. The temperature during the chemical reaction II-2a is not particularly limited and may be the temperature during addition of the organic base, and for example, from -10°C to 10°C. The pressure during the chemical reaction II-2a is, for example, from 0.1 to 1 MPa, preferably from 0.1 to 0.5 MPa, and more preferably 0.1 MPa. The time required for the chemical reaction II-2a is not particularly limited and is, for example, from 0.5 to 24 hours. The chemical reaction II-2a is preferably carried out under an inert gas atmosphere.

Subsequently, the reaction liquid containing the compound (5) obtained in the chemical reaction II-2a can be used directly to carry out the chemical reaction II-2b.

Examples of the alkali metal t-butoxide that can be used in the chemical reaction II-2b include lithium t-butoxide, sodium t-butoxide, and potassium t-butoxide.

The amount of alkali metal t-butoxide used in the chemical reaction II-2b is, for example, preferably from 1.0 to 2.5 moles, and more preferably from 1.0 to 1.5 moles, with respect to 1 mole of the compound (3).

The addition of alkali metal t-butoxide is preferably carried out gradually in order to suppress a sudden temperature rise due to the heat of reaction. The addition rate of the alkali metal t-butoxide may be adjusted by monitoring the temperature inside a reactor. The temperature during the addition of the alkali metal t-butoxide is preferably from -10°C to 10°C. The temperature during the chemical reaction II-2b is not particularly limited and may be the temperature during the addition of the alkali metal t-butoxide, or a higher temperature, and for example, from -10°C to 50°C. The pressure during the chemical reaction II-2b is, for example, from 0.1 to 1 MPa, preferably from 0.1 to 0.5 MPa, and more preferably 0.1 MPa. The time required for the chemical reaction II-2b is not particularly limited and is, for example, from 0.5 to 24 hours. The chemical reaction II-2b is preferably carried out under an inert gas atmosphere.

After completion of the chemical reaction II-2b, post-treatments such as extraction with an organic solvent, and drying and concentration of the obtained organic layer can be performed. Furthermore, if necessary, a product in the chemical reaction II-2b may be purified by operations such as recrystallization and chromatography.

### [Conversion of compound (6) to compound (3) (chemical reaction I)]

Although the compound (3) as the substrate used in the chemical reaction 11-1 or II-2 is not particularly limited by its production method, for example, it can be obtained by a method including conversion from the compound (6) to the compound (3).

The compound (6) as the substrate in the chemical reaction I is a compound represented by the formula (6).

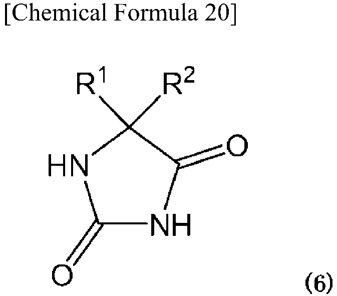

In the formula (6), R¹ and R² are the same as those defined in the formula (1).

The compound (6) used in the present embodiment may be synthesized by oneself through a known method, or may be synthesized through some form of method and made commercially available by others.

A compound (7) is used in the chemical reaction I. The compound (7) is a compound represented by formula (7).

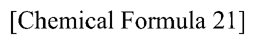

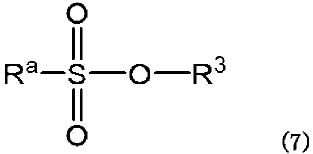

In the formula (7), R³ is the same as that defined in the formula (1). R^{a} represents a methyl group, a trifluoromethyl group, a phenyl group, or a tolyl group.

In the present embodiment, R^{a} is preferably a phenyl group.

The compound (7) used in the present embodiment may be synthesized by oneself through a known method, or may be synthesized through some form of method and made commercially available by others.

An inorganic base can be used in the chemical reaction I. Examples of the inorganic base include sodium carbonate, sodium bicarbonate, potassium carbonate, potassium bicarbonate, sodium hydroxide, potassium hydroxide, sodium hydride, and potassium hydride.

The amount of the compound (6) with respect to 1 mole of the compound (7) in the chemical reaction I is not particularly limited, but is, for example, from 0.6 to 2.5 moles, preferably from 1.0 to 2.0 moles, and more preferably from 1.0 to 1.5 moles.

The amount of the inorganic base used in the chemical reaction I is, for example, preferably from 1.0 to 2.0 moles, more preferably from 1.0 to 1.5 moles, with respect to 1 mole of the compound (7).

An organic solvent can be used in the chemical reaction I. The organic solvent that can be used in the chemical reaction I is not particularly limited as long as it is inert with respect to the chemical reaction I. Examples of the organic solvent that can be used in the chemical reaction I include ethers such as diethyl ether, diisopropyl ether, diethylene glycol dimethyl ether (product name: diglyme), tetrahydrofuran (abbreviated name: THF), 4-methyltetrahydropyran (abbreviated name: MTHP), and dioxane; halogenated hydrocarbons such as dichloromethane, chloroform, and 1,2-dichloroethane (abbreviated name: DCE); aliphatic hydrocarbons such as pentane, hexane, cyclohexane, heptane, and octane; aromatic hydrocarbons such as toluene and xylene; esters such as methyl acetate, ethyl acetate, propyl acetate, butyl acetate, dimethyl carbonate, and diethyl carbonate; ketones such as acetone, anone, and methyl isobutyl ketone (abbreviated name: MIBK); and other solvents such as dimethyl sulfoxide (abbreviated name: DMSO) and dimethylformamide (abbreviated name: DMF). One type of these organic solvents can be used alone or two or more types thereof can be used in combination. The amount of the organic solvent used is not particularly limited, but is preferably from 1 to 100 parts by mass with respect to 1 part by mass of the compound (7).

One embodiment of the chemical reaction I involves mixing the compound (6), the compound (7), an inorganic base, and an organic solvent to complete the chemical reaction.

The temperature during mixing is not particularly limited and may be room temperature. The temperature during the chemical reaction I is, for example, from 60°C to 120°C. The pressure during the chemical reaction I is, for example, from 0.1 to 1 MPa, preferably from 0.1 to 0.5 MPa, and more preferably 0.1 MPa. The time required for the chemical reaction I is not particularly limited, but is, for example, from 0.5 to 24 hours. The chemical reaction I is preferably carried out under an inert gas atmosphere.

After completion of the chemical reaction I, the reaction liquid is added to an acidic aqueous solution to neutralize any remaining inorganic base. Then, post-treatments such as extraction with an organic solvent, and drying and concentration of the obtained organic layer can be performed. Furthermore, if necessary, a product in the chemical reaction I may be purified by operations such as recrystallization and chromatography.

### Examples

The present invention will be described in more detail below with reference to Examples. The present invention is not limited by the following Examples.

### [Example 1]

### Synthesis of 5,5-dimethyl-3-(2,2,2-trifluoroethyl)imidazolidine-2,4-dione (compound 3)

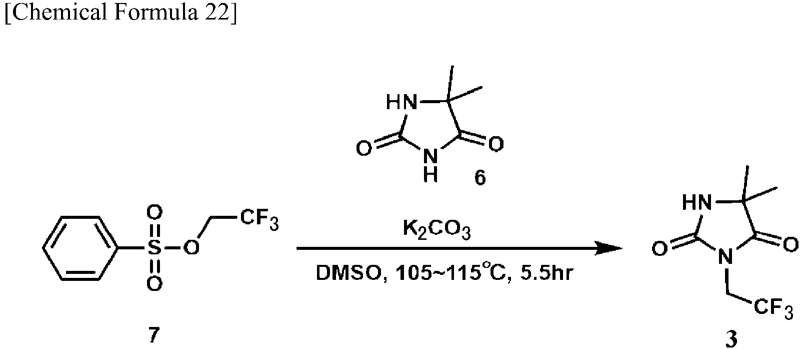

A compound 7 (2,2,2-trifluoroethyl benzenesulfonate, 118.9 g, 0.50 mol), a compound 6 (5,5-dimethylimidazolidine-2,4-dione, 96.1 g, 1.5 eq.), potassium carbonate (71.0 g, 1.0 eq.), and dimethyl sulfoxide (500 mL, 1.0 L/mol) were charged into a reactor and stirred at 105 to 115°C for 5.5 hours. After that, water (500 mL), toluene (500 mL), and 35 wt% hydrochloric acid (52.1 g) were added and mixed. The resulting mixture was separated, and an aqueous layer was extracted twice with toluene (500 mL) and separated to obtain a toluene layer. NaCl (10.0 g) and toluene (500 mL) were added to the aqueous layer obtained by separation, and the resulting mixture was heated to 50°C, and then separated to obtain a toluene layer. The obtained toluene layers were combined into one, and then toluene was removed by distillation under reduced pressure to obtain a dimethyl sulfoxide solution of compound 3 at a yield of 74%. The aqueous layer contained the compound 3 at a yield of 17%.

The dimethyl sulfoxide solution of compound 3 was purified by silica gel column chromatography to obtain the compound 3. The ¹H-NMR data and ¹³C-NMR data for the obtained compound 3 are shown below.

¹H-NMR(CDCl₃/TMS, δ (ppm)) 6.33 (brs, 1H), 4.16-4.10 (q, 2H), 1.49 (s, 6H)
¹³C-NMR(CDCl₃/TMS, δ (ppm)) 176.45, 154.98, 127.28-118.94 (q), 59.29, 40.07-38.98 (q), 24.73

### [Example 2]

### Synthesis of tert-butyl 5,5-dimethyl-2,4-dioxo-3-(2,2,2-trifluoroethyl)imidazolidine-1-carboxylate (compound 1)

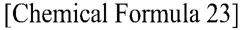

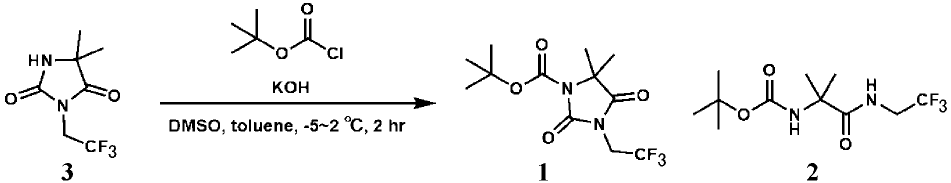

The dimethyl sulfoxide solution of Compound 3 obtained in Example 1 (2.87 g, purity: 74.5 wt%, 10.2 mmol), toluene (2.0 mL, 0.2 L/mol), and dimethyl sulfoxide (3.4 mL, 0.3 L/mol) were charged into a reactor, 85% KOH pellets (2.64 g, 3.9 eq.) were added thereto while stirring at 25°C, followed by stirring at 25 to 30°C for 40 minutes. The resulting mixture was cooled to -2°C, and a separately prepared tert-butyl carbonochloridate/toluene solution (14.06 g, purity: 19.4 wt%, 2.0 eq.) was dropwise added thereto over 15 minutes at -2 to 0°C, followed by stirring at -5 to 2°C for 2 hours to obtain a reaction liquid. After that, 5 wt% hydrochloric acid (10 mL) was charged into a flask and cooled, and the reaction liquid was dropwise added thereto over 10 minutes at -2 to 1°C. After completion of the dropwise addition, the resulting mixture was stirred at 0 to 20°C for 1 hour. A toluene layer was obtained by separation. A toluene solution of compound 1 was obtained at a yield of 87%. This toluene solution also contained the compound 2 at a yield of 9%.

Toluene was removed from the toluene solution of compound 1 by distillation to obtain the compound 1 (3.28 g, purity: 80.61 wt%, 8.53 mmol) in a yellow oil form. This was purified by silica gel column chromatography to obtain the compound 1.

The ¹H-NMR data and ¹³C-NMR data for the obtained compound 1 are shown below.

¹H-NMR (CDCl₃/TMS, δ (ppm)) 4.21-4.14 (q, 2H), 1.67 (s, 6H), 1.58 (s, 9H)
¹³C-NMR (CDCl₃/TMS, δ (ppm)) 173.91, 150.48, 148.14, 127.03-118.69 (q), 84.76, 63.51, 40.36-39.26 (q), 28.04, 23.06

### [Example 3]

### Synthesis of tert-butyl (2-methyl-1-oxo-1-((2,2,2-trifluoroethyl)amino)propan-2-yl)carbamate (compound 2)

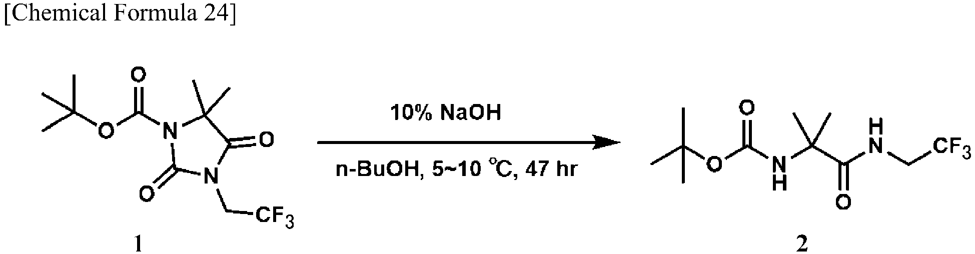

Toluene was removed by distillation from the toluene solution of compound 1 obtained in Example 2 to obtain the compound 1 (3.28 g, purity: 80.61 wt%, 8.53 mmol) in a yellow oil form. This was charged into a reactor. n-butanol (18.8 mL, 2.2 L/mol) was added thereto for dissolution, cooled to 7°C, and 10 wt% NaOH (15.02 g, 4.4 eq.) was added, and the resulting mixture was stirred at 5 to 10°C for 24 hours. Subsequently, 10 wt% NaOH (7.51 g, 2.2 eq.) was added thereto, and the resulting mixture was further stirred at 5 to 10°C for 23 hours. Water (9.6 mL) was then added thereto, and the resulting mixture was neutralized to pH 5.7 with 35 wt% hydrochloric acid (5.78 g, 6.5 eq.). Water (9.6 mL) and n-butanol (24.0 mL) were then added thereto, and the resulting mixture was stirred at 30 to 40°C for 30 minutes. After that, an n-butanol layer was obtained by separation. An n-butanol solution of compound 2 was obtained at a yield of 84%. n-butanol was partially removed from this solution by distillation under reduced pressure to obtain a slurry of compound 2. This was filtered to obtain the compound 2 as white crystals.

The ¹H-NMR data and ¹³C-NMR data for the obtained compound 2 are shown below.

¹H-NMR (CDCl₃/TMS, δ (ppm)) 7.22 (brs, 1H), 4.82 (brs, 1H), 3.96-3.87 (dq, 2H), 1.50 (s, 6H), 1.44 (s, 9H)
¹³C-NMR (CDCl₃/TMS, δ (ppm)) 175.04, 155.05, 128.31-120.03 (q), 80.88, 56.99, 41.35-40.32, 28.18, 25.62

### [Example 4]

### Synthesis of tert-butyl 5,5-dimethyl-2,4-dioxo-3-(2,2,2-trifluoroethyl)imidazolidine-1-carboxylate (compound 1)

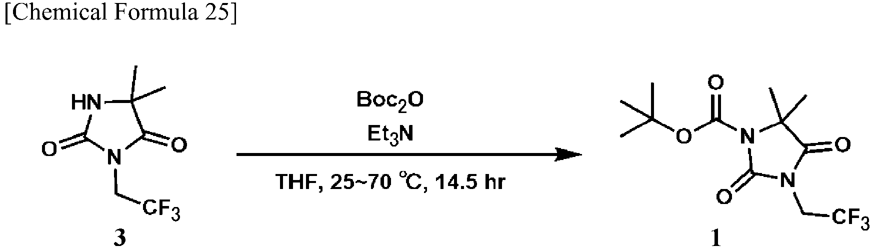

The compound 3 (0.63 g, 3.0 mmol), tetrahydrofuran (2.5 mL, 0.8 L/mol), and triethylamine (0.61 g, 2.0 eq.) were charged into a reactor, and (Boc)₂O (di-tert-butyl dicarbonate, 0.69 g, 1.1 eq.) dissolved in tetrahydrofuran (0.5 mL) was dropwise added thereto while stirring at 25°C. After completion of the dropwise addition, the resulting mixture was stirred at 25 to 50°C for 1.5 hours, followed by stirring at 70°C for 13 hours. During the above stirring, triethylamine (0.30 g, 1.0 eq.) and (Boc)₂O (di-tert-butyl dicarbonate, 0.16 g, 0.2 eq.) were added. Water (6.0 mL), toluene (6.0 mL), and 35 wt% hydrochloric acid (0.72 g) were then added thereto, mixed, and separated. The organic layer was washed twice with 5 wt% hydrochloric acid (5.0 mL), washed once with water (5.0 mL), dehydrated over magnesium sulfate, filtered, and the organic solvent was removed by distillation under reduced pressure to obtain the compound 1 at a yield of 95%.

### [Example 5]

### Synthesis of tert-butyl 5,5-dimethyl-2,4-dioxo-3-(2,2,2-trifluoroethyl)imidazolidine-1-carboxylate (compound 1)

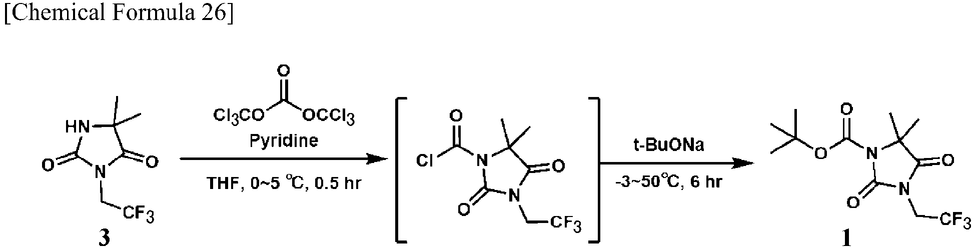

The compound 3 (1.09 g, purity: 96.3 wt%, 50 mmol), tetrahydrofuran (5.0 mL, 1.0 L/mol), and triphosgene (0.74 g, 0.5 eq.) were charged into a reactor and stirred at 5°C, and pyridine (0.59 g, 1.5 eq.) was dropwise added thereto. After completion of the dropwise addition, the resulting mixture was stirred at 0 to 5°C for 0.5 hours. After that, the resulting mixture was cooled to -1°C, t-BuONa (0.48 g, 1.0 eq.) was added thereto, and the resulting mixture was stirred at -3 to 10°C for 3.5 hours, then at 25 to 50°C for 1.5 hours. Subsequently, t-BuONa (0.24 g, 0.5 eq.) was added thereto and the resulting mixture was stirred at 50°C for 1 hour. Water (10 mL), toluene (5.0 mL), and 28 wt% NaOH (0.85 g) were added thereto, and the resulting mixture was separated to obtain a toluene solution of compound 1 at a yield of 15%.

### [Comparative Example 1]

### Hydrolysis of compound 3

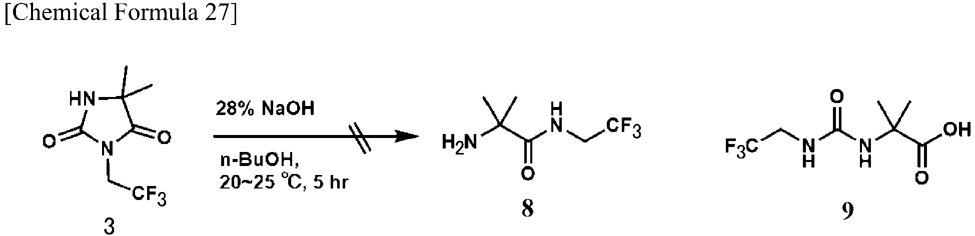

The compound 3 (0.22 g, purity: 96.3 wt%, 1.0 mmol) and n-butanol (1.0 mL, 1.0 L/mol) were charged into a flask, and 28 wt% NaOH (0.29 g, 2.0 eq.) was added thereto while stirring, and the resulting mixture was stirred at 20 to 25°C for 5 hours. After that, water (2.0 mL) and n-butanol (1.0 mL) were added thereto and mixed, and the resulting mixture was separated. An n-butanol solution of compound 9 (yield: 39%) and an aqueous solution of compound 9 (yield: 60%) were obtained. n-butanol was removed from the n-butanol solution of compound 9 by distillation under reduced pressure to obtain the compound 9.

The ¹H-NMR data for the obtained compound 9 are shown below.

¹H-NMR (DMSO-d₆/TMS, δ (ppm)) 7.26-7.23 (brt, 1H), 6.83 (s, 1H), 3.77-3.68 (dq, 2H), 1.36 (s, 6H)

### [Comparative Example 2]

### Hydrolysis of compound 1'

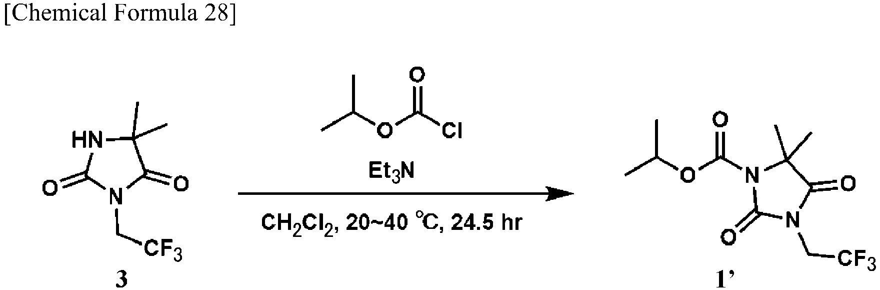

The compound 3 (0.63 g, 3.0 mmol), dichloromethane (3.0 mL, 1.0 L/mol), and triethylamine (0.33 g, 1.1 eq.) were added to a flask, and isopropyl carbonochloridate (0.40 g, 1.1 eq.) was dropwise added thereto while stirring. After completion of the dropwise addition, the resulting mixture was stirred at 20 to 40°C for 4.5 hours. Subsequently, triethylamine (0.33 g, 1.1 eq.) and isopropyl carbonochloridate (0.40 g, 1.1 eq.) were added thereto, and the resulting mixture was stirred at 40°C for 20 hours. Chloroform (5.0 mL), water (5.0 mL), and 35 wt% hydrochloric acid (0.39 g) were added thereto and mixed, and the resulting mixture was separated. The organic layer was washed with water (5.0 mL) and separated to obtain a chloroform solution of compound 1' at a yield of 59%. The chloroform solution of compound 1' was dehydrated by adding magnesium sulfate and filtered, the organic solvent was removed by distillation under reduced pressure, and the resulting mixture was purified by silica gel column chromatography to obtain the compound 1'. The ¹H-NMR data for the obtained compound 1' are shown below.

¹H-NMR (CDCl₃/TMS, δ (ppm)) 5.19-5.10 (sep, 1H), 4.21-4.15 (q, 2H), 1.68 (s, 6H), 1.40-1.38 (d, 6H)

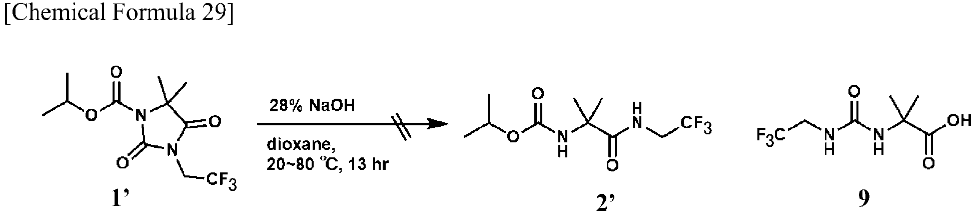

The compound 1' (0.10 g, purity: 81 wt%, 0.27 mmol) and 1,4-dioxane (0.32 mL, 1.2 L/mol) were charged into a flask, and 28 wt% NaOH (0.04 g, 1.0 eq.) was added thereto while stirring. The resulting mixture was stirred at 20 to 50°C for 7 hours and then at 80°C for 6 hours. 28 wt% NaOH (0.24 g, 6.0 eq.) was added during the above stirring. Water (4.0 mL) and chloroform (4.0 mL) were added thereto and mixed, and the resulting mixture was separated. An aqueous solution of compound 9 was obtained at a yield of 80%. The compound 2' was not detected in either the chloroform layer or the aqueous layer.

### [Example 6]

### Synthesis of hydrochloride salt of 2-amino-2-methyl-N-(2,2,2-trifluoroethyl)propanamide (compound 8)

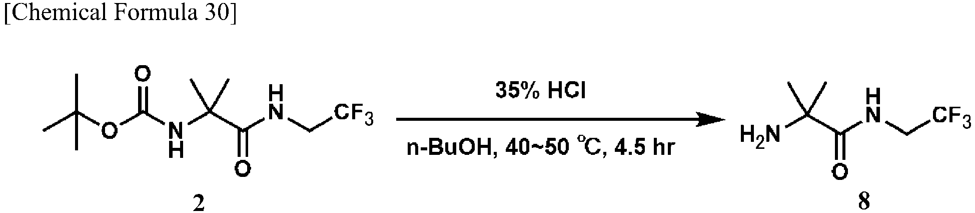

A slurry of compound 2 (1.92 g, 6.76 mmol) in n-butanol (18.3 mL, 2.7 L/mol) and 35 wt% hydrochloric acid (3.81 g, 5.4 eq.) were charged into a flask, and the resulting mixture was stirred at 40 to 50°C for 4.5 hours. After that, toluene (30.1 mL) and water (13.1 mL) were added thereto and mixed, and the resulting mixture was separated. An aqueous solution of a hydrochloride salt of compound 8 was obtained at a yield of 97%.

### [Example 7]

### Synthesis of 2-amino-2-methyl-N-(2,2,2-trifluoroethyl)propanamide (compound 8)

Chloroform (5.0 mL) was added to an aqueous solution of a hydrochloride salt of compound 8 (7.37 g, purity: 5.90 wt%, 2.36 mmol) obtained in Example 6 and stirred. 28 wt% NaOH (1.75 g, 5.2 eq.) was added thereto for neutralization to a pH of 11.3, and the resulting mixture was separated. The aqueous layer was extracted twice with chloroform (3.0 mL) and separated. The organic layers were combined into one to obtain a chloroform solution of compound 8 with a distribution rate of 98%. Chloroform was removed by distillation under reduced pressure to obtain the compound 8.

The ¹H-NMR data and ¹³C-NMR data for the obtained compound 8 are shown below.

¹H-NMR (CDCl₃/TMS, δ (ppm)) 8.15 (brs, 1H), 3.92-3.83 (dq, 2H), 1.46 (brs, 2H), 1.38 (s, 6H)
¹³C-NMR (CDCl₃/TMS, δ (ppm)) 178.04, 128.39-120.08 (q), 54.99, 41.18-40.15 (q), 29.08

### INDUSTRIAL APPLICABILITY

A novel method for producing an N-BOC amino acid amide compound can be provided.

## Claims

1. A method for producing a compound represented by formula (2), the method comprising hydrolyzing a compound represented by formula (1): 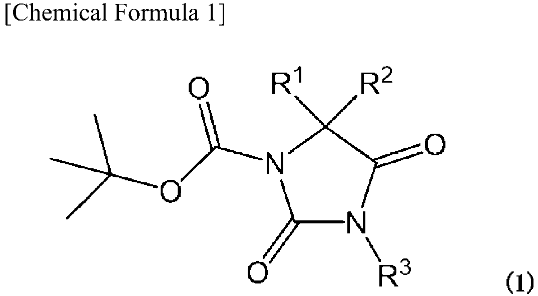
wherein R¹ and R² each independently represent a hydrogen atom, a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₃₋₆ cycloalkyl group, a substituted or unsubstituted phenyl group, or a substituted or unsubstituted 5- or 6-membered heteroaryl group; and R¹ and R² may together form a substituted or unsubstituted C₂₋₅ alkylene group,
R³ represents a substituted or unsubstituted C₁₋₆ alkyl group, a substituted or unsubstituted C₂₋₆ alkenyl group, a substituted or unsubstituted C₂₋₆ alkynyl group, a substituted or unsubstituted C₃₋₆ cycloalkyl group, a substituted or unsubstituted phenyl group, or a substituted or unsubstituted 5- or 6-membered heteroaryl group, 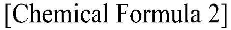 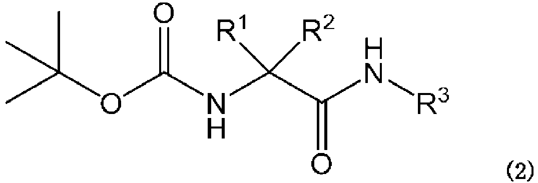
wherein R¹, R², and R³ are the same as those defined in the formula (1).

2. The method according to Claim 1, further comprising chemically reacting a compound represented by formula (3) with a compound represented by formula (4) to obtain the compound represented by the formula (1), 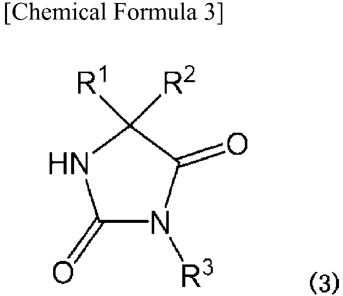 wherein R¹, R², and R³ are the same as those defined in the formula (1), 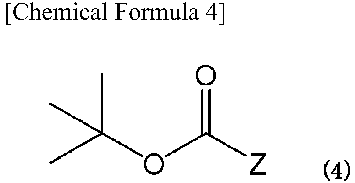 wherein Z represents a t-butoxycarbonyloxy group, a halogeno group, or a 1H-imidazol-1-yl group.

3. The method according to Claim 1, further comprising: chemically reacting a compound represented by formula (3) with phosgene, diphosgene, or triphosgene to obtain a compound represented by formula (5); and
chemically reacting the compound represented by the formula (5) with an alkali metal t-butoxide to obtain the compound represented by the formula (1), 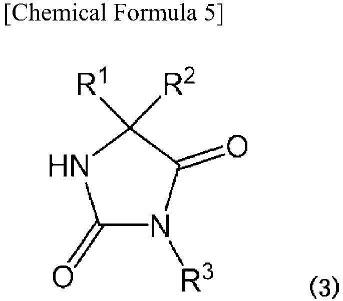
wherein R¹, R², and R³ are the same as those defined in the formula (1), 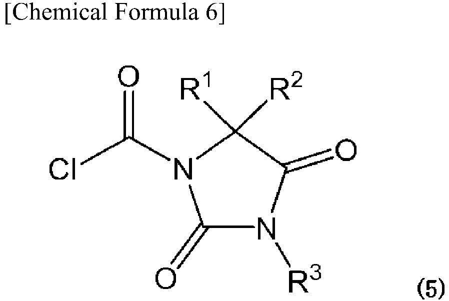
wherein R¹, R², and R³ are the same as those defined in the formula (1).

4. The method according to Claim 2 or 3, further comprising chemically reacting a compound represented by formula (6) with a compound represented by formula (7) to obtain the compound represented by the formula (3), 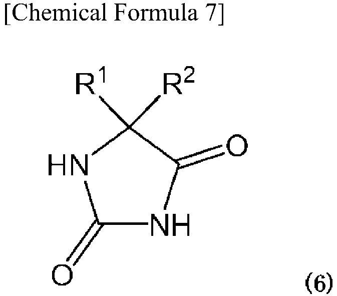 wherein R¹ and R² are the same as those defined in the formula (1), 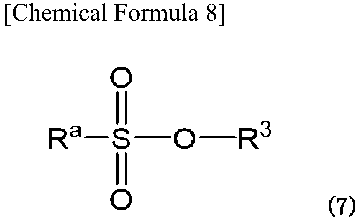 wherein R³ is the same as that defined in the formula (1), R^{a} represents a methyl group, a trifluoromethyl group, a phenyl group, or a tolyl group.
